# EUROPÄISCHE PATENTSCHRIFT

(11) **EP 1 303 231 B1**
(45) Veröffentlichungstag und Bekanntmachung des Hinweises auf die Patenterteilung: **27.10.2004**
(21) Anmeldenummer: 01953655.6
(22) Anmeldetag: 24.07.2001
(51) Int. Cl.: A61F 2/01

(54) **VASKULÄRE VERANKERUNGSEINRICHTUNG**
VASCULAR ANCHORING DEVICE
DISPOSITIF D'ANCRAGE VASCULAIRE

(30) Priorität: 25.07.2000 AT 20001305
(43) Veröffentlichungstag der Anmeldung: 23.04.2003
(73) Patentinhaber: Petrovic, Dragan, 1120 Wien (AT); Vujanic, Aleksandar, 1200 Wien (AT); Brenner, Werner, 1190 Wien (AT); Hassan, Ali, 1210 Wien (AT)
(72) Erfinder: Petrovic, Dragan, 1120 Wien (AT); Vujanic, Aleksandar, 1200 Wien (AT); Brenner, Werner, 1190 Wien (AT); Hassan, Ali, 1210 Wien (AT)
(74) Vertreter: Haffner, Thomas M., Dr.
(86) Internationale Anmeldenummer: PCT/AT2001/000253
(87) Internationale Veröffentlichungsnummer: WO 2002/007649

(56) Entgegenhaltungen:
- WO-A-00/07521
- FR-A- 2 580 504
- FR-A- 2 666 980

## Beschreibung

Die Erfindung bezieht sich auf eine vaskuläre Verankerungseinrichtung für die Einführung in ein Blutgefäß mittels eines Katheters, mit in radialer Richtung aufweitbaren Spreizen.

Zur Positionierung von intravaskulären Eingriffswerkzeugen oder Hilfsmitteln für die Behandlung werden üblicherweise Katheter verwendet, mit welchen durch die Positionierung von Ballons eines Ballonkatheters die Aufweitung von Blutgefäßen ermöglicht wird oder Stents oder chirurgische Werkzeuge für chirurgische Eingriffe in Blutgefäße eingebracht werden können. Der Katheter wird hierbei in aller Regel über ein Blutgefäß, beispielsweise die Arteria femoralis oder brachialis eingebracht, um an einer entsprechenden Stelle die erforderliche Behandlung vornehmen zu können. Während der Intervention besteht aber immer die Gefahr, daß von der Gefäßwand Partikel, insbesondere Plaquepartikel, in die Blutbahn freigesetzt werden und auf diese Weise Schäden verursachen, insbesondere embolisieren und somit die Blutversorgung für das zielgewebe unterbrechen oder drosseln. Es wurde in diesem Zusammenhang daher bereits vorgeschlagen, Filtersysteme einzusetzen, mit welchen es gelingt, während der Intervention freigesetzte Partikel abzufangen. Bekannte Filtersysteme, wie sie beispielsweise in der US-A-5,300,086 beschrieben sind, weisen in radialer Richtung aufweitbare Spreizen auf, zwischen welchen ein Filtermaterial in radialer Richtung aufgespannt werden kann. Bei dieser bekannten Einrichtung sind die Spreizen in radialer Richtung vorgespannt ausgebildet und werden von einem Hüllschlauch, welcher mit dem Führungsdraht und dem Filter eingebracht wird, in eingeklappter Position gehalten. Durch Zurückziehen des Hüllschlauches gelingt es in der Folge, die Spreizen unter der Kraft der Vorspannung nach außen zu bewegen. Bei einer Festlegung derartiger Spreizen in einem Blutgefäß läßt sich allerdings der Führungsdraht nicht mehr frei bewegen, und insbesondere in axialer Richtung über die Stelle nicht hinausbewegen, in welcher sich die radial aufweitbaren Spreizen in Kontakt mit der Gefäßwand befinden. Die Spreizkraft ist durch die elastische Vorspannung der Spreizen begrenzt. Für das Einfahren bzw. Einklappen dieser radial aufgeweiteten Spreizen kann ein schlauchartiger Applikator wiederum über die Spreizen übergestülpt werden, um die Einwärtsbewegung entgegen der Kraft der Vorspannung bzw. entgegen der Federkraft der Spreizen zu bewirken. Während das Öffnen der Spreizen durch eine Zugbewegung vorgenommen wird, erfolgt das Schließen durch eine Schiebebewegung des Betätigungsgliedes in entgegengesetzter Richtung.

Weiters ist aus der FR 2 580 504 A eine Einrichtung zur Positionierung eines intravaskulären Filters bekanntgeworden. Bei dieser Einrichtung wird ein über Katheter und Führungsdraht einführbarer und mittels eines aufblasbaren Ballons reversibel aufweitbarer Blutfilter beschrieben, welcher in einer Vene verankerbar ist. Der Blutfilter besteht aus mehreren flexiblen Armen.

Die Erfindung zielt nun darauf ab, eine zurückziehbare vaskuläre Verankerungseinrichtung der eingangs genannten Art zu schaffen, mit welcher das Öffnen und Schließen jeweils mit einer in die gleiche Richtung erfolgenden Zugbewegung erfolgen kann, und weiters sichergestellt ist, daß nach einer definierten Auswärtsbewegung und Verankerung der Spreizen eine weitestgehend freie Beweglichkeit des Führungsdrahtes über die Verankerungsstelle hinaus gewährleistet ist.

Zur Lösung dieser Aufgabe besteht die erfindungsgemäße Verankerungseinrichtung im wesentlichen darin, daß die Verankerungseinrichtung aus einem im wesentlichen hohlzylindrischen Teil mit von einer Stirnseite ausgehenden sich axial erstreckenden Spreizen und einem koaxial im hohlzylindrischen Teil an einem Führungsdraht des Katheters geführten Verriegelungsglied besteht, daß das Verriegelungsglied an seinem den Spreizen zugewandten Ende wenigstens eine Auflauffläche trägt, welche durch axiale Verschiebung relativ zu den Spreizen eine Aufweitung der Spreizen bewirkt und wenigstens einen in der Spreizstellung mit dem hohlzylindrischen Teil zusammenwirkenden Anschlag trägt und daß der Führungsdraht einen weiteren Anschlag aufweist, welcher bei Verschieben des Führungsdrahtes mit einer Gegenanschlagsfläche des Verriegelungsgliedes oder einer Anschlagfläche des hohlzylindrischen Teiles zusammenwirkt. Dadurch, daß die Verankerungseinrichtung aus einem im wesentlichen hohlzylindrischen Teil mit von einer Stirnseite ausgehenden, sich axial erstreckenden Spreizen und einem koaxial im hohlzylindrischen Teil an einem Führungsdraht des Katheters geführten Verriegelungsglied besteht, wird die Möglichkeit geschaffen, durch eine Relativverschiebung des Verriegelungsgliedes relativ zu dem die Spreizen tragenden Teil, beispielsweise durch einfaches Zurückziehen des Führungsdrahtes, eine Auswärtsbewegung der Spreizen zu erzielen, wofür die Ausbildung so getroffen ist, daß das koaxial am Führungsdraht verschiebliche Verriegelungsglied an seinem den Spreizen zugewandten Ende wenigstens eine Auflauffläche trägt, welche durch axiale Verschiebung relativ zu den Spreizen eine Aufweitung der Spreizen bewirkt. Zu diesem Zweck ist es lediglich erforderlich, den die Spreizen tragenden, hohlzylindrischen Teil entsprechend gegen eine Verschiebung zu sichern, was durch einen Applikator in Form eines über den Führungsdraht aufgeschobenen Schlauches in einfacher Weise gelingt. Durch Zurückziehen des Führungsdrahtes gelangt ein Anschlag des Führungsdrahtes in Wirkverbindung mit dem Verriegelungsglied und verschiebt das Verriegelungsglied relativ zum hohlyzylindrischen Teil, wodurch aufgrund der Auflaufflächen eine Aufweitung der Spreizen bewirkt wird. In kinematischer Umkehr dieser Bewegung kann ein Anschlag des Führungsdrahtes mit dem die Spreizen tragenden Bauteil zusammenwirken, wobei der Applikator mit dem Verriegelungsglied zusammenwirkt. Dadurch, daß nun das Verriegelungsglied wenigstens einen in der Spreizstellung mit dem hohlzylindrischen Teil zusammenwirkenden Anschlag trägt, kann das Verriegelungsglied in eine entsprechende Position zurückgezogen werden, in welcher die Spreizen eine definierte Spreizstellung einnehmen und gleichzeitig eine Verriegelung der Spreizstellung erfolgt, da der Anschlag ein neuerliches Verschieben des Verriegelungsgliedes entgegen der Verschieberichtung des Führungsdrahtes verhindert. Derartige Anschläge können von beliebig geformten in Achsrichtung miteinander zusammenwirkenden Flächen, wie z.B. Nuten, Löchern, Federn oder dgl. gebildet sein. In diesem Zustand tritt somit eine selbsttätige Verriegelung ein, und der Führungsdraht behält seine freie axiale Verschiebbarkeit zumindest in dem Ausmaß, in welchem der Anschlag des Führungsdrahtes nicht mit dem Verriegelungsglied bzw. dem die Spreizen tragenden Bauteil kollidiert. Für ein selbsttätiges Schließen der aufweitbaren Spreizen bzw. eine Lösung der Verankerung kann nun in der Folge durch neuerliches Aufbringen des schlauchartigen Applikators durch Bewegung in die gleiche Richtung das Verriegelungsglied über die Verriegelungsposition hinaus zurückgezogen werden, wobei sich aufgrund der Elastizität des hohlzylindrischen Teiles ein selbsttätiges Einwärtsbewegen der sich im wesentlichen axial erstreckenden, aber radial auswärts bewegten Spreizen wiederum ergibt. Die Einwärtsbewegung kann in besonders vorteilhafter Weise dadurch erleichtert werden, daß, wie es einer bevorzugten Ausbildung der Erfindung entspricht, die Spreizen von einem Ring aus elastomerem Material umgriffen sind. Die Aufweitung bzw. das Ausfahren der Spreizen in radialer Richtung kann dadurch begünstigt werden, daß die Auflaufflächen als Keil- oder Konusflächen ausgebildet sind.

Bei einer derartigen Ausbildung ergibt sich somit eine Kinematik, bei welcher beispielsweise durch Zurückziehung des Führungsdrahtes unter Kollision des Anschlages des Führungsdrahtes mit dem Verriegelungsglied zunächst ein Aufweiten der Spreizen über die Auflaufflächen, welche als Keil-, Ringwulst- oder Konusflächen ausgebildet sein können, erfolgt, und in der weiteren Folge durch weiteres Zurückziehen des Führungsdrahtes, das heißt durch gleichsinnige Bewegung, unter Mitnahme des Verriegelungsgliedes eine Einwärtsbewegung der Spreizen ausgelöst wird.

Die Verankerungsvorrichtung kann für beliebige Zwecke eingesetzt werden und bietet den wesentlichen Vorteil, daß sie unter Aufrechterhalten der vollen Funktionalität des Führungsdrahtes eine definierte Ankerstelle bietet, relativ zu welcher bzw. an welcher weitere Hilfsmittel angeordnet werden können. Am Führungsdraht können in der Folge Ballons eines Ballonkatheters oder Stents oder chirurgische Instrumente an die Behandlungsstelle für den intravaskulären Eingriff eingebracht werden, wobei die Verankerungseinrichtung in besonders einfacher Weise naturgemäß auch zur Aufspannung eines Filters herangezogen werden kann.

Zusätzliche Behandlungshilfsmittel, wie beispielsweise Einrichtungen zur dosierten Abgabe von Medikamenten können mit der Verankerungseinrichtung in einfacher Weise gekoppelt werden, wofür die Ausbildung mit Vorteil so getroffen ist, daß das den Spreizen abgewandte Ende des hohlzylindrischen Teiles Kupplungsglieder, insbesondere magnetische oder verrastbare Kupplungsglieder trägt. Derartige Kupplungsglieder, welche eine lösbare Kupplung und damit auch ein Entkuppeln ermöglichen, erlauben auch eine entsprechende Dislozierung des Ankers bei eingefahrenen Spreizen mittels geeigneter kuppelbarer Applikatoren.

Um das Einbringen der Spreizen und das zurückziehen der wiederum eingefahrenen Spreizen zu erleichtern, ist die Ausbildung bevorzugt so getroffen, daß die Spreizen als an den hohlzylindrischen Teil anschließende, in Achsrichtung geknickte oder gewölbte Finger ausgebildet sind.

Insgesamt wird somit eine positionierbare und lösbare vaskuläre Verankerungseinrichtung geschaffen, welche sich mit besonders einfachen Mitteln an der gewünschten Stelle einbringen und festlegen läßt, wobei das Einbringen und das Ausbringen ohne Bewegungsrichtungsumkehr des Führungsdrahtes, beispielsweise mit einer einfachen Zugbewegung, gelingt. Gleichzeitig wird in der positionierten Stellung der Verankerungseinrichtung eine weitestgehende Beweglichkeit und Verschieblichkeit des Führungsdrahtes für nachfolgende Operationen gewährleistet, welche intravaskuläre Eingriffe wesentlich erleichtert. Die intravaskuläre Behandlung kann den Standards entsprechend im Blutgefäß über über einen Führungsdraht eingeführte Ballons und/oder Stents od. dgl. erfolgen, wobei bei Verwendung der Verankerungseinrichtung als Filter gleichzeitig während der Intervention freigesetzte Partikel abgefangen werden können. Die Verankerungseinrichtung kann in besonders vorteilhafter Weise aus Materialien ausgebildet werden, die unter Röntgenstrahlung sichtbar sind und beispielsweise metallische Filterfinger umfassen, sodaß die jeweilige Position sicher verfolgt werden kann.

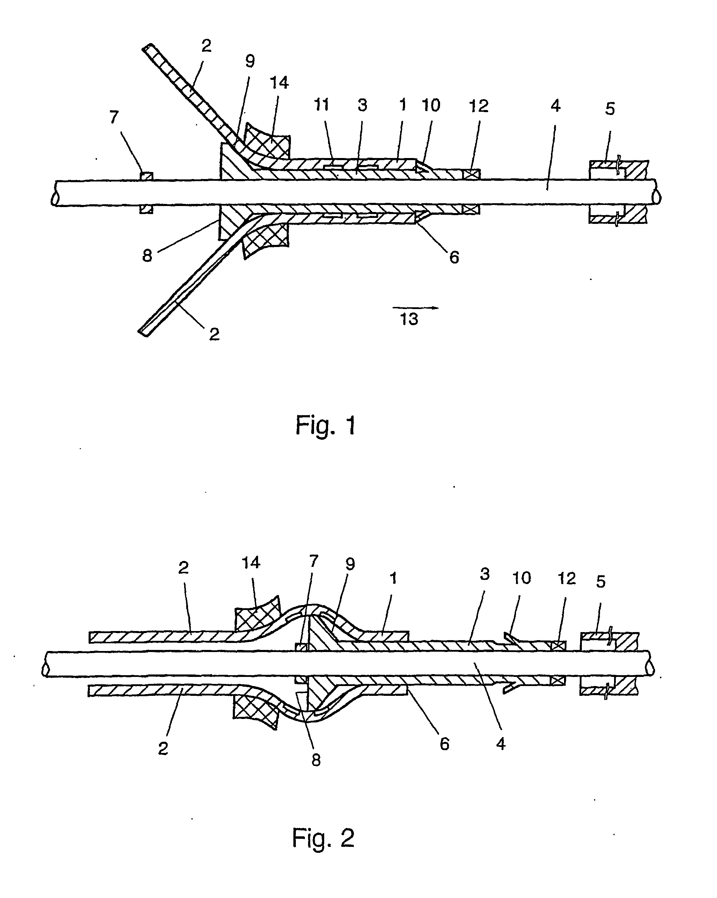

Die Erfindung wird nachfolgend anhand eines in der Zeichnung schematisch dargestellten Ausführungsbeispieles näher erläutert. In dieser zeigen Fig.1 einen Querschnitt durch die erfindungsgemäße Verankerungseinrichtung mit ausgefahrenen Spreizen und Fig.2 einen Querschnitt anlog der Fig.1 bei eingeklappten Spreizen.

In Fig.1 ist mit 1 ein im wesentlichen zylindrischer Grundkörper bezeichnet, an dessen Stirnseite Spreizen 2 vorgesehen sind. Der im wesentlichen zylindrische Grundkörper 1 ist gemeinsam mit einem Verriegelungsglied 3 jeweils konzentrisch zu einem Führungsdraht 4 in Achsrichtung desselben verschiebbar geführt. Das Einbringen erfolgt über einen schematisch mit 5 angedeuteten Applikator, welcher nach Art eines flexiblen Schlauches über den Führungsdraht 4 geschoben wird und auf diese Weise mit der den Spreizen 2 abgewandten Stirnfläche 6 des hohlzylindrischen Teiles 1 zusammenwirkt. In eingeklappter Position der Spreizen kann somit mittels des Applikators 5 der hohlzylindrische Teil 1 mit den Spreizen in die jeweilige Position gebracht werden. Durch Zurückziehen des Führungsdrahtes 4 gelingt es, einen am Führungsdraht angeordneten Anschlag 7 mit der Stirnfläche 8 oder einem in Achsrichtung vorragenden Anschlag des Verriegelungsteiles 3 in Wirkverbindung zu bringen, wodurch der Verriegelungsteil 3 unter Aufweitung der Spreizen 2 zurückgezogen wird. Die Aufweitung der Spreizen 2 erfolgt hierbei über als Konusflächen ausgebildete Auflaufflächen 9 des Verriegelungsteiles. Der Verriegelungsteil 3 trägt wenigstens einen elastisch ausklappbaren oder verformbaren Anschlag 10, welcher in verschiedenen Positionen mit dem hohlzylindrischen Teil 1 zusammenwirken kann. Zu diesem Zweck sind Ringnuten 11 vorgesehen, wobei der elastisch verformbare Anschlag 10 jeweils in einer dieser Ringnuten 11 verrasten kann und auf diese Weise eine bestimmte Spreizstellung der Spreizen 2 zur Folge hat. Anstelle von Ringnuten 11 können Durchbrechungen, wie z.B. Schlitze oder Löcher, vorgesehen sein, wobei in kinematischer Umkehr naturgemäß Ringnuten, Schlitze oder Löcher im Verriegelungsteil 3 mit entsprechenden elastisch verformbaren Anschlägen 10 am hohlzylindrischen Teil 1 zusammenwirken können. Bei vollständig ausgefahrenen Spreizen hintergreift der dargestellte ausklappbare Ausschlag 10 die den Spreizen abgewandte Stirnfläche 6 des hohlzylindrischen Teiles, wobei eine Arretierung in voneinander verschiedenen Spreizstellungen der Spreizen 2 erfolgen kann. Nach der Arretierung und dem Ausfahren der Spreizen 2 kann der Applikator 5 wieder abgezogen werden und der Führungsdraht 4 in axialer Richtung verschoben werden, wobei diese Verschiebung lediglich dadurch begrenzt ist, daß der Anschlag 7 des Führungsdrahtes 4 nicht über den Anschlag 8 des Verriegelungsgliedes 3 bei positionierter Verankerungseinrichtung zurückgezogen werden kann.

Der Verriegelungsteil 3 kann an seinem den Spreizen abgewandten Ende schematisch mit 12 bezeichnete Kupplungsglieder tragen, an welchen weitere Einrichtungen verankert werden können.

Zum Entriegeln der Verankerungseinrichtung kann der Applikator 5 neuerlich aufgeschoben werden und mit der Stirnfläche 6 des hohlzylindrischen Teiles zusammenwirken. Wenn in der Folge der Führungsdraht 4 in Richtung des Pfeiles 13 zurückgezogen wird, gelangt der die Auflaufflächen 9 tragende Teil unter elastischer Verformung des hohlzylindrischen Teiles 1 in den hohlzylindrischen Teil, wodurch sich die Spreizen 2 wiederum radial einwärts bewegen und außer Eingriff mit der Gefäßwand gelangen. Diese Einwärtsbewegung kann durch einen Elastomerring 14, wie er insbesondere in Fig.2 ersichtlich ist, begünstigt werden. In dieser einwärts geklappten Position der Spreizen 2 gelingt es, die verankerungseinrichtung ohne Gefahr einer Läsion des Blutgefäßes wiederum vollständig zurückzuziehen.

Mit den Spreizen 2 kann ein in den Figuren nicht dargestellter Filter verbunden sein, welcher bei der in Fig.1 dargestellten Position der Spreizen über den gesamten Querschnitt des Blutgefäßes aufgespannt werden kann.

## Patentansprüche

1. Vaskuläre Verankerungseinrichtung für die Einführung in ein Blutgefäß mittels eines Katheters, mit in radialer Richtung aufweitbaren Spreizen, **dadurch gekennzeichnet, daß** die Verankerungseinrichtung aus einem im wesentlichen hohlzylindrischen Teil (1) mit von einer Stirnseite ausgehenden sich axial erstreckenden Spreizen (2) und einem koaxial im hohlzylindrischen Teil (1) an einem Führungsdraht (4) des Katheters geführten Verriegelungsglied (3) besteht, daß das Verriegelungsglied (3) an seinem den Spreizen (2) zugewandten Ende wenigstens eine Auflauffläche (9) trägt, welche durch axiale Verschiebung relativ zu den Spreizen (2) eine Aufweitung der Spreizen (2) bewirkt und wenigstens einen in der Spreizstellung mit dem hohlzylindrischen Teil (1) zusammenwirkenden Anschlag (10) trägt und daß der Führungsdraht (4) einen weiteren Anschlag (7) aufweist, welcher bei Verschieben des Führungsdrahtes (4) mit einer Gegenanschlagsfläche (8) des Verriegelungsgliedes (3).

2. Vaskuläre Verankerungseinrichtung nach Anspruch 1, **dadurch gekennzeichnet, daß** die Auflauffläche (9) als Keil-, Ringwulst- oder Konusfläche ausgebildet ist.

3. Vaskuläre Verankerungseinrichtung nach Anspruch 1 oder 2, **dadurch gekennzeichnet, daß** die Spreizen (2) von einem Ring (14) aus elastomerem Material umgriffen sind.

4. Vaskuläre Verankerungseinrichtung nach Anspruch 1, 2 oder 3, **dadurch gekennzeichnet, daß** das den Spreizen (2) abgewandte Ende des hohlzylindrischen Teiles (1) Kupplungsglieder (12), insbesondere magnetische oder verrastbare Kupplungsglieder trägt.

5. Vaskuläre Verankerungseinrichtung nach einem der Ansprüche 1 bis 4, **dadurch gekennzeichnet, daß** mit den Spreizen (2) ein auf den aufgeweiteten Querschnitt aufspannbares Filter verbunden ist.

6. Vaskuläre Verankerungseinrichtung nach einem der Ansprüche 1 bis 5, **dadurch gekennzeichnet, daß** die Spreizen (2) als an den hohlzylindrischen Teil (1) anschließende, in Achsrichtung geknickte oder gewölbte Finger ausgebildet sind.

## Claims

1. A vascular anchoring device to be introduced into a blood vessel by the aid of a catheter and including struts capable of being expanded in the radial direction, **characterized in that** the anchoring device is comprised of a substantially hollow-cylindrical portion (1) including axially extending struts (2) departing from an end face, and a locking member (3) guided coaxially within the hollow-cylindrical portion (1) on a guiding wire (4) of the catheter, that the locking member (3), on its end facing the struts (2), carries at least one run-up surface (9) which causes the expansion of the struts (2) by axial displacement relative to the struts (2) and carries at least one stop (10) cooperating with the hollow-cylindrical portion (1) in the expanded position, and that the guiding wire (4) comprises a further stop (7) cooperating with a counter stop surface (8) of the locking member (3) upon displacement of the guiding wire (4).

2. A vascular anchoring device according to claim 1, **characterized in that** the run-up surface (9) is designed as a wedge surface, torus surface or cone surface.

3. A vascular anchoring device according to claim 1 or 2, **characterized in that** the struts (2) are embraced by a ring (14) made of elastomer material.

4. A vascular anchoring device according to claim 1, 2 or 3, **characterized in that** the end of the hollow-cylindrical portion (1), that faces the struts (2) carries coupling members (12) and, in particular, magnetic or latchable coupling members.

5. A vascular anchoring device according to any one of claims 1 to 4, **characterized in that** a filter capable of being clamped on the expanded cross section is connected with the struts (2).

6. A vascular anchoring device according to any one of claims 1 to 5, **characterized in that** the struts (2) are designed as fingers following the hollow-cylindrical potion (1) and bent or curved in the axial direction.

## Revendications

1. Dispositif d'ancrage vasculaire pour l'introduction dans un vaisseau sanguin au moyen d'un cathéter, comportant des écarteurs pouvant être élargis dans la direction radiale, **caractérisé en ce que** le dispositif d'ancrage est constitué d'une partie (1) sensiblement cylindrique creuse avec des écarteurs (2) s'étendant axialement depuis une face frontale, et d'un organe de verrouillage (3) guidé coaxialement dans la partie (1) cylindrique creuse sur un fil de guidage (4) du cathéter, **en ce que** l'organe de verrouillage (3) porte, à son extrémité tournée vers les écarteurs (2), au moins une surface de butée (9) qui provoque, par coulissement axial par rapport aux écarteurs (2), un élargissement des écarteurs (2), et porte au moins une butée (10) coopérant dans la position écartée avec la partie (1) cylindrique creuse, et **en ce que** le fil de guidage (4) comporte une autre butée (7) qui, pendant le coulissement du fil de guidage (4), coopère avec une contre-surface de butée (8) de l'organe de verrouillage (3).

2. Dispositif d'ancrage vasculaire selon la revendication 1, **caractérisé en ce que** la surface de butée (9) est réalisée comme surface en coin, en bourrelet annulaire ou en cône.

3. Dispositif d'ancrage vasculaire selon la revendication 1 ou 2, **caractérisé en ce que** les écarteurs (2) sont entourés par un anneau (14) en matériau élastomère.

4. Dispositif d'ancrage vasculaire selon la revendication 1, 2 ou 3, **caractérisé en ce que** l'extrémité, tournée à l'opposé des écarteurs (2), de la partie (1) cylindrique creuse, porte des organes d'accouplement (12), en particulier des organes d'accouplement magnétiques ou à encliquetage.

5. Dispositif d'ancrage vasculaire selon l'une des revendications 1 à 4, **caractérisé en ce qu'**un filtre, pouvant être tendu sur la section transversale élargie, est relié aux écarteurs (2).

6. Dispositif d'ancrage vasculaire selon l'une des revendications 1 à 5, **caractérisé en ce que** les écarteurs (2) sont réalisés en tant que doigts pliés ou bombés dans la direction axiale, se raccordant à la partie (1) cylindrique creuse.
